# EUROPEAN PATENT APPLICATION

(11) **EP 3 922 205 A1**
(43) Date of publication of application: **15.12.2021**
(21) Application number: 20179850.1
(22) Date of filing: 12.06.2020
(51) Int. Cl.: A61B 50/00, A61B 50/20, A61B 50/30, A61B 50/33, A61B 50/22, B65G 1/00, B65G 37/00, B65G 47/90

(54) **SYSTEM FOR ARRANGING CONTAINERS AND CONTAINER LIDS ON A RACK IN A STERILE PROCESSING DEPARTMENT**

(71) Applicant: Gibotech A/S, 5220 Odense SØ (DK)
(72) Inventor: Anker, Henrik, 5220 Odense SØ (DK)
(74) Representative: AWA Denmark A/S

(57) **Abstract**

A system for arranging containers and container lids on a rack. The system comprises a first rotation unit for receiving the rack, a second rotation unit for conveying the rack, and a robotic arm configured to arrange containers and container lids conveyed by the one or more motorized conveyor elements onto the rack received by the first rotation unit. The first rotation unit is configured to rotate the rack received relative to the robotic arm around a first vertical axis, to allow the robotic arm to arrange containers on the rack, and to convey the rack received to the second rotation unit. The second rotation unit is configured to rotate the rack around a second vertical axis, and to convey the rack to one or more motorized conveyor elements configured for conveying the rack, or one or more transport vehicles configured for transporting the rack.

## Description

### Field

The present disclosure relates to a system for arranging containers and container lids on a rack in a sterile processing department, and to a sterile processing department comprising a system for arranging containers and container lids on a rack.

### Background

Increasing healthcare cost is a global problem which every country face. Automation of manual process in hospitals can be part of the solution. However, due the high complexity of hospitals the degree of automation until now has been low.

Cleaning and sterilizing of medical equipment such as endoscopes and surgical instruments in a sterile processing department is an example of a work intensive process. However, the work may be hazardous as medical equipment may be contaminated with medical risk, which may pose health risks for personnel needing to handle the contaminated medical equipment. Thereby, lowering the human interaction needed with contaminated equipment is desirable.

Furthermore, the procedures for handling contaminated medical equipment are complicated. As strict requirements and protocols for correct handling and of the medical equipment are present, to minimize contamination and to increase the safety of personnel working with the contaminated medical equipment.

Thus it remains a problem to provide a simpler and safer system that can aid in automating processes in a sterile processing department and further improve a work environment for personnel.

### Summary

According to a first aspect, the disclosure relates to a system for arranging containers and container lids on a rack comprising a first rack side and a second rack side in a sterile processing department, said sterile processing department comprising one or more motorized conveyor elements configured for conveying the containers and container lids, and one or more transport vehicles configured for transporting the rack, where the system comprises:
- a first rotation unit for receiving the rack,
- a second rotation unit for conveying the rack, and
- a robotic arm configured to arrange containers and container lids conveyed by the one or more motorized conveyor elements onto the rack received by the first rotation unit,
wherein the first rotation unit is configured to:
- rotate the rack received relative to the robotic arm around a first vertical axis, thereby allowing the robotic arm to arrange containers on both the first rack side and the second rack side of the rack received, and
- convey the rack received to the second rotation unit
wherein the second rotation unit is configured to:
- rotate the rack received from the first rotation unit around a second vertical axis, and
- convey the rack received from the first rotation unit to one or more motorized conveyor elements configured for conveying the rack, or one or more transport vehicles configured for transporting the rack.

Consequently, a system with a high through-put for efficient stacking of containers in a sterile environment is achieved. The system allows stacking of containers with minimal to no human interaction reducing contamination risk of both containers and users interacting with the containers. Furthermore, automation of the stacking of containers may lessen the work cost and personnel associated with handling of containers in the sterile processing department. Being able to rotate racks in the system and stack different rack sides allows for full utilization of the racks.

The rack may be any structure capable of receiving containers on at least two different rack sides. The rack may also be configured to receive containers on more than two different rack sides, e.g. three rack sides or four rack sides. In an embodiment where the rack comprises three or four rack sides, the first rotation unit may be configured to rotate the rack around the first vertical axis to allow the robotic arm to stack each of the rack sides. Each rack side may be adapted to comprise several racks levels configured for receiving one or more containers. The rack may comprise an identifier allowing identification and/or tracking of the rack. The identifier may be a barcode, a RFID, and/or a QR-code.

The robotic arm, the first rotation unit and/or the second rotation unit may comprise a processing unit. The processing unit may be any unit which comprises a unit able to perform basic arithmetic, such as a central processing unit (CPU) or a graphics processing unit (GPU). The processing unit may be able to communicate with each other, by providing the processing units with a transceiver, which allows the transmittal and receiving of information.

The robotic arm may comprise sensors and/or cameras in order to orientate itself and/or to assess distances. The robotic arm may comprise force sensors allowing the robotic arm to assess if contact have been made with a container, and/or to assess if a container has been properly stacked onto the rack. The robotic arm may comprise means for lifting the containers, such as suction means, gripper element, and/or mechanical means being configured for lifting the lid of the container. The suction means may be suction cups. The gripper element may be any element capable of gripping a container and holding the container to allow the robotic arm to move the container. The mechanical means may be a fork capable of engaging the container on a lower side of the container and lifting the container, other mechanical means capable of engaging the container may also be used. The robotic arm may be capable of movement in both the vertical plane and the horizontal plane, thereby giving a highly flexible robotic arm. The robotic arm may be a modular unit not integrally connected with other units in the system. Having the robotic arm as a modular unit may be preferable as easy replacement or repair of the robotic arm is possible in case of failure or malfunction. For example, the robotic arm may be configured to be mounted directly to a floor of a sterile processing department.

The first rotation unit may be any unit which is capable of rotating the rack, and conveying the rack. The first rotation unit may be configured to rotate the rack by having the rack situated on a rotational platform of the first rotation unit. The rotational platform of the first rotation unit being able to rotate around a first vertical axis, thereby rotating a rack on the rotational platform around the first vertical axis. Alternatively, the first rotation unit may be provided with means for lifting and rotating the rack. The first rotation unit may be provided with a motorized roller(s), belt conveyor or chain conveyor for conveying the rack. The motorized conveyor elements may be used in conjunction with non-motorized conveyor elements such as non-motorized roller elements. The first rotation unit may be a modular unit not integrally connected with other units in the system. Having the first rotation unit as a modular unit may be preferable as easy replacement or repair of the first rotation unit is possible in case of failure or malfunction.

The second rotation unit may be any unit which is capable of rotating the rack, and conveying the rack. The second rotation unit may be configured to rotate the rack by having the rack situated on a rotational platform of the second rotation unit. The rotational platform of the second rotation unit being able to rotate around a second vertical axis, thereby rotating a rack on the rotational platform around the second vertical axis. Alternatively, the second rotation unit may be provided with means for lifting and rotating the rack. The second rotation unit may be provided with a motorized roller(s), belt or chain conveyor for conveying the rack. The motorized conveyor elements may be used in conjunction with non-motorized conveyor elements such as non-motorized roller elements. The second rotation unit may be a modular unit not integrally connected with other units in the system. Having the second rotation unit as a modular unit may be preferable as easy replacement or repair of the second rotation unit is possible in case of failure or malfunction.

The first rotation unit, the second rotation unit, and/or the robotic arm may comprise one or more position sensors configured for detecting a position of the rack. Alternatively, the one or more position sensors is one or more external position sensors, e.g. ceiling mounted cameras monitoring the rack. The one or more position sensors may be used for assuring the rack have reached a stacking position, allowing the robotic arm to stack containers onto the rack. The one or more position sensors may be used for tracking the rack, assuring the rack moves as planned through the system. The one or more positions sensors may be communicatively connectable to a processing unit of the first rotation unit, the second rotation unit, and/or the robotic arm. The one or more position sensors may be provided with transmitters capable of transmitting collected position data. The one or more position sensors may be optical, mechanical, inductive, and/or electrical sensors.

The one or more motorized conveyor elements may be a motorized roller(s), belt or chain conveyor. The motorized conveyor elements may be used in conjunction with non-motorized conveyor elements such as non-motorized roller elements.

The container may be a sterilizing container, e.g. a container configured to support the medical equipment in an autoclave. The sterilizing container may be provided with a lid allowing a sterilizing fluid e.g. hot steam, to enter the container. The lid may further be configured to form a fluid tight seal when the sterilizing container cools down whereby the medical equipment within the medical container may be stored under sterile conditions.

The one or more transport vehicles may be autonomous transport vehicles or user driven transport vehicles. The transport vehicle may follow markers or wires in the floor, lasers or any other guiding means for navigation. The transport vehicle may also be fully configured to travel without any guiding means e.g. by having a pre-programmed travelling path or by having a map of the navigation area for determining the travelling path based on the map and the navigation means. The transport vehicle may comprise a motorized roller, belt, or chain conveyor for delivering the container.

In an embodiment the system further comprises a first outgoing unit, the first outgoing unit comprising one or more motorized conveyor elements, wherein the first outgoing unit is configured to:
- receive the rack conveyed by the second rotation unit, and
- convey the rack in a first conveyor direction to a second outgoing unit, or the one or more transport vehicles configured for transporting the rack.

Providing a first outgoing unit allows for buffering of loaded racks, i.e. one loaded rack may be on the second rotation unit and another rack may be on the first outgoing unit, thereby allowing the system to hold two loaded racks ready for being taken away. The first outgoing unit may comprise a motorized roller(s), belt or chain conveyor for conveying the rack. The motorized conveyor elements may be used in conjunction with non-motorized conveyor elements such as non-motorized roller elements. The system may also comprise a second outgoing unit, a third outgoing unit, a fourth outgoing unit, and so forth, allowing for customization of buffering of loaded racks. In some embodiments the first outgoing unit may also be configured to hold more than one loaded rack, e.g. two loaded racks, three loaded racks, four loaded racks, and so forth. The first outgoing unit may be a modular unit not integrally connected with other units in the system. Having the first outgoing unit as a modular unit may be preferable as easy replacement or repair of the first outgoing unit is possible in case of failure. In the context of the invention a loaded rack is to be understood as a rack on which a container has been stacked by the robotic arm.

In an embodiment the system further comprises a first ingoing unit, the first ingoing unit comprising one or more motorized conveyor elements, wherein the first ingoing unit is configured to:
- receive the rack from a second ingoing unit or the one or more transport vehicles configured for transporting the rack.
- convey the rack in a second conveyor direction to the first rotation unit.

Providing a first ingoing unit allows for buffering of empty racks, i.e. one empty rack may be on the first rotation unit and another empty rack may be on the first ingoing unit, thereby allowing the system to hold two empty racks ready for being stacked with containers. The first ingoing unit may comprise a motorized roller(s), belt or chain conveyor for conveying the rack. The motorized conveyor elements may be used in conjunction with non-motorized conveyor elements such as non-motorized roller elements. The system may also comprise a second ingoing unit, a third ingoing unit, a fourth ingoing unit, and so forth, allowing for customization of buffering of empty racks. In some embodiments the first ingoing unit may also be configured to hold more than one empty rack, e.g. two empty racks, three empty racks, four empty racks, and so forth. The first ingoing unit may be a modular unit not integrally connected with other units in the system. Having the first ingoing unit as a modular unit may be preferable as easy replacement or repair of the first ingoing unit is possible in case of failure. In the context of the invention an empty rack is to be understood as a rack on which the robotic arm has not stacked a container.

In an embodiment the first conveyor direction and the second conveyor direction are parallel to each other.

Thereby, a compact stacking system with buffer capabilities for both empty and loaded racks may be achieved. Having the first conveyor direction and the second conveyor direction in parallel to each other, assures the first outgoing unit and the first ingoing unit can be placed in a space efficient manner, to assure the system have as small of a footprint as possible.

In an embodiment the system further comprises a waste collector for collecting waste from the container and lids before being arranged on the rack.

The waste collector may be any container suitable for containing medical waste. The waste collector may comprise a drain and piping leading to a waste reservoir. The robotic arm may be configured to hold and/or move the containers above the waste collector to allow waste from the containers to fall into the waste collector before being stacked onto the rack. In some embodiments the robotic arm is configured to hold the container over the waste collector for a predetermined time, before stacking the container on the rack. Having a waste collector may assure the spread of waste within the sterilizing department is minimized, furthermore the risk of waste from the containers interacting negatively, e.g. short circuiting electronic or clogging up passages, with the first rotation unit and the second rotation unit is also lowered.

In an embodiment the first rotation unit, and the second rotation unit, each comprises a conveyor unit for conveying the rack, and wherein each of the conveyor units are substantially identical.

Having the conveyor units being substantially identical allows for the same spare parts to be used for both the first rotation unit and the second rotation unit. Furthermore, only a single type of replacement unit needs to be kept on hand in case of failure of the conveying unit, thereby reducing the complexity of logistics and storage of spare units. In an embodiment where the first rotation unit and the second rotation unit also each comprises a rotational platform, the rotational platform may also be substantially identical. In an embodiment wherein the system comprises one or more outgoing units and/or one or more in going units, said one or more outgoing units and/or one or more in going units may also each comprise a conveyor unit substantially identical to the conveyor unit of the first rotation unit and/or the second rotation unit.

In an embodiment each conveyor unit is a belt conveyor.

Using belt conveyors may assure a high degree of precision in conveying of the racks is achieved. This may allow for precise movement of the rack relative to the robotic arm, which may be advantageous if the rack is to be placed in a specific stacking position, before being able to be stacked. Furthermore, each belt conveyor may comprise a scraper arrangement for scraping waste of the belts. Such scraper arrangements may assure there is no built-up of waste on the conveyor belts.

In an embodiment the belt conveyors of the first rotation unit and the second rotation unit comprises waste liners for shielding the respective belt conveyor from waste.

Providing waste liners may assure waste from the containers does not interact with internal part of the belt conveyors, which may possibly lead to failure or malfunction. The liners may be polymer inserts, metallic inserts, or ceramic inserts.

In embodiment the first rotation unit and the second rotation unit each comprises a first cushion and a second cushion configured to delimit movement of the rack in a non-conveying direction, and where the first cushion and the second cushion are opposed to each other.

Providing the first rotation unit and the second rotation unit with cushions may help in assuring the rack does not tip over or fall off the first rotation unit or the second rotation unit. Furthermore, as the cushions delimit movement in a non-conveying direction, the cushions also assure the orientation of the rack does not change unnecessarily while being conveyed, i.e. the rack does not rotate in an unplanned manner, which may lead to the rack getting stuck. The cushions may be formed from polymer, metal, or ceramic.

In an embodiment the first cushion and the second cushion each extends longitudinally with a first longitudinal end and a second longitudinal end, and wherein the first cushion and the second cushion taper toward their respective first longitudinal end and second longitudinal end.

Having the cushions extending longitudinally enables the cushion to also acts as guides for the rack while being conveyed, assuring the rack is conveyed, received and handed-off in an optimal manner. Furthermore, the tapering at the longitudinal ends of the cushions form a funnel, which may ease the receiving and conveying of racks between units in the system.

In an embodiment the first rotation unit and the second rotation unit each comprises a rotational platform configured to rotate the rack, and wherein the first rotation unit and the second rotation unit each further comprises a waste shield, where the waste shield is arranged to be in-between the respective rotational platform and the rack when the first rotation unit and the second rotation unit receives the rack.

Having a waste shield shielding off the rotational platform from waste, minimizes risk of waste interacting with the rotational platform and potentially leading to failure or malfunction. Furthermore, having the rack rotating on a rotational platform may assure the racks movement relative to the first rotation unit and the second rotation unit when rotated is minimized. Minimizing the racks movement relative to the first rotation unit and the second rotation unit minimizes the risk of misaligning the rack or wrongfully orientating the rack.

According to a second aspect the disclosure relates to use of a system disclosed in relation to the first aspect of the disclosure in a sterile processing department.

According to a third aspect the disclosure relates to a sterile processing department comprising a system disclosed in relation to the first aspect of the disclosure.

In an embodiment the sterile processing department further comprises one or more motorized conveyor elements configured for conveying containers and container lids, one or more transport vehicles configured for transporting the rack, and a washing machine for washing containers and container lids on the rack, wherein the washing machine is configured for receiving the rack from the one or more transport vehicles.

The different aspects of the present invention can be implemented in different ways including a transport system, use of a transport system and a sterile processing department described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependent claims. Furthermore, it will be appreciated that embodiments described in connection with one of the aspects described herein may equally be applied to the other aspects.

### Brief description of the drawings

The above and/or additional objects, features and advantages of the present invention, will be further elucidated by the following illustrative and non-limiting detailed description of embodiments of the present invention, with reference to the appended drawings, wherein:
Figure 1 is a perspective view of an embodiment of a system according to the invention.
Figure 2 is a perspective view of an embodiment of a first rotation unit of the system according to the invention.
Figure 3 is a perspective view of an embodiment of a second rotation unit of the system according to the invention.
Figure 4 is a perspective view of an embodiment of a second outgoing unit of the system according to the invention.
Figure 5 is a perspective view of an embodiment of a first ingoing unit of the system according to the invention.
Figure 6 is a perspective view of an embodiment of a robotic arm and a waste collector of the system according to the invention.

### Detailed description

In the following description, reference is made to the accompanying figures, which show by way of illustration how the invention may be practiced.

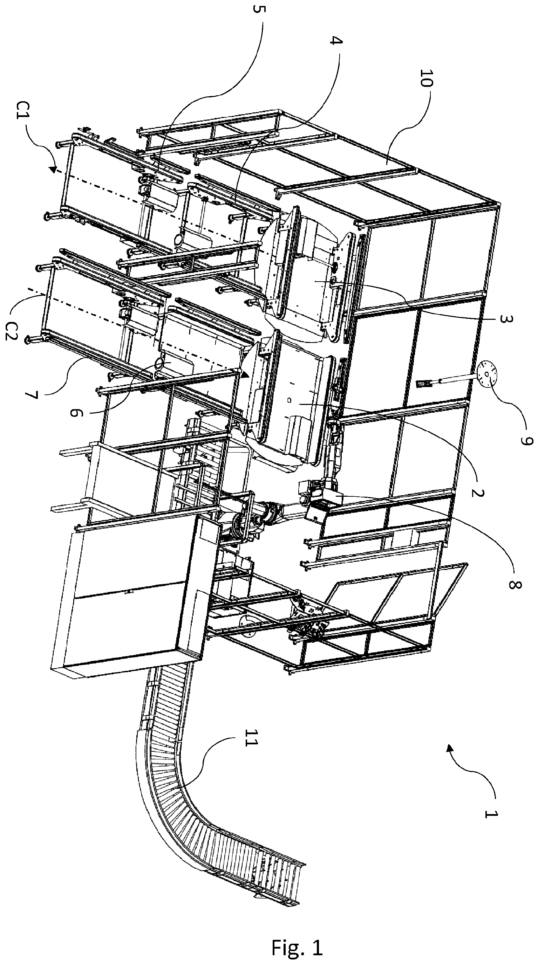

Referring initially to figure 1, which shows a perspective view of an embodiment of a system 1 according to the invention. The system 1 comprises a first ingoing unit 6 and a second ingoing unit 7. The second ingoing unit 7 is configured for receiving a rack from a motorized vehicle or one or more motorized conveyor elements. After receiving the rack, the second ingoing unit 7 is configured for conveying the rack along a second conveyor direction C2 to the first ingoing unit 6. The first ingoing 6 unit is configured for receiving the rack from the second ingoing unit 7 and to convey it along the second conveyor direction C2 to a first rotation unit 2. The first rotation unit 2 is configured for receiving the rack from the first ingoing unit 6. The first rotation unit 2 is configured for rotating the rack around a first vertical axis to allow a robotic arm 8 to stack at least a first rack side and a second rack side of the rack with containers and lids. The containers and lids are conveyed to the robotic arm 8 by one or more motorized conveyor elements 11, which in the shown embodiment is a plurality of rollers 11. When the containers and lids have been conveyed to the robotic arm 8, the robotic arm 8 is configured for stacking the containers and lids onto the first rack side and the second rack side of the rack received on the first rotation unit 2. After the rack have been stacked with containers and lids, the first rotation unit 2 is configured to convey the rack to a second rotation unit 3. The second rotation unit 3 is configured for receiving the rack from the first rotation unit 2 and rotating the rack around a second vertical axis. The second rotation unit 3 is configured to convey the rack to a first outgoing unit 4. The first outgoing unit 4 is configured for receiving the rack from the second rotation unit 3 and to convey it along a first conveyor direction to a second outgoing unit 5. The second outgoing unit 5 is configured for receiving the rack from the first rotation unit 4 and to convey along the first conveyor direction C1 to a motorized vehicle or one or more motorized conveyor elements. The first conveyor direction C1 is parallel to the second conveyor direction C2, allowing for a compact set-up of the outgoing units 4 and 5 and the ingoing units 6 and 7. The system 1 is monitored by a sensor 9. The sensor may be configured to detect a position of the rack and/or detect if the rack(s) are moving as planned through-out the system 1. The sensor 9 may be a camera unit 9. The sensor 9 may be communicatively connected to another unit of the system 1 via a transmitter in the sensor 9 and receiver in the unit to which it needs to communicate. The system 1 is partly enclosed by a barrier 10 to shield off and to protect the system 1 from external factors. The barrier 10 is preferably formed by a plurality of modular walls, which allows easy rearrangement and set-up of the barrier 10.

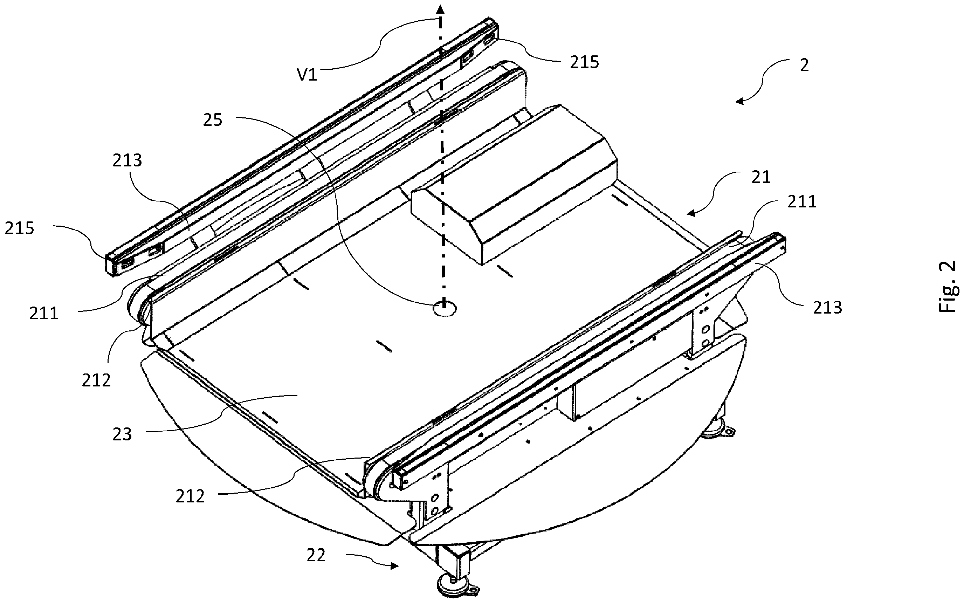

Referring to figure 2, which is a perspective view of an embodiment of a first rotation unit 2 of the system 1 according to the invention. The first rotation unit 2 comprises a conveyor unit 21. The conveyor unit 21 is configured for receiving and conveying a rack. The conveyor unit 21 comprises belt conveyors 211 for conveying the rack. Other conveying means, such as motorized rollers or chain conveyors may also be used. The belt conveyors 211 are provided with waste liners 212 for protecting internal electronics and mechanics of the belt conveyors 211. The waste liners 212 reduces the risk of malfunctioning and/or failure of the belt conveyors 211. In the shown embodiment the conveyor unit 21 comprises a first cushion and a second cushion 213. The first cushion and the second cushion 213 are used to delimit movement of a rack in non-conveying directions and to guide the rack along conveying directions. The first cushion and the second cushion 213 extends longitudinally with a first longitudinal end and a second longitudinal end. The first cushion and the second cushion 213 taper towards their respective first longitudinal end and second longitudinal end. The tapering of the cushions 213 leads to the cushions forming a funnel at each longitudinal end, facilitating easy receiving and conveying of racks. The first cushion and the second cushion 213 comprises cavities 215. The cavities 215 may comprise position sensors. In an embodiment the cavities 215 comprise one or more lasers and one or more receivers, where one or more lasers is placed within the cavities 215 of either the first cushion and/or the second cushion 213, and in the cavities 215 opposing the lasers in the opposing cushion 213 are receivers configured for registering light emitted by the lasers. This provides one or more simple position sensors for tracking a position of a rack, since whenever the receiver does not register light emitted by the laser it corresponds to a rack passing by the laser. The conveyor unit 21 is connected to a rotational platform 22. The rotational platform 22 is capable of rotating around a first vertical axis V1. The rotation of the rotational platform 22 leads to a rotation of the conveyor unit around the first vertical axis V1. The rotational platform 22 comprises height adjustable legs, for either lowering or raising the rotational platform 22 and the conveyor unit 21. Arranged in-between the rotational platform 22 and the conveyor unit 21 is a waste shield 23. The waste shield is arranged to protect the rotational platform 22 from waste dripping of containers and/or lids arranged or being arranged on the rack. The waste shield 23 comprises a through-going hole 24. The through-going hole 24 may facilitate correct alignment of the rotation unit 2 in relation to the robotic arm 8, when setting-up the first rotation unit 2. In some embodiments a downwards extending hollow cylinder extends from the rim of the through-going hole 24. The hollow cylinder may be configured to go into engagement with a structure extending upwards from a floor of a sterile processing department. This may keep the rotation unit 2 locked in-place and may further facilitate correct alignment relative to the robotic arm. Preferably a center point of the through-going hole intersects the first vertical axis V1, and a center axis of the hollow cylinder corresponds to the first vertical axis V1. The first rotation unit 2 is a modular unit and may be moved independently of other parts within the system 1.

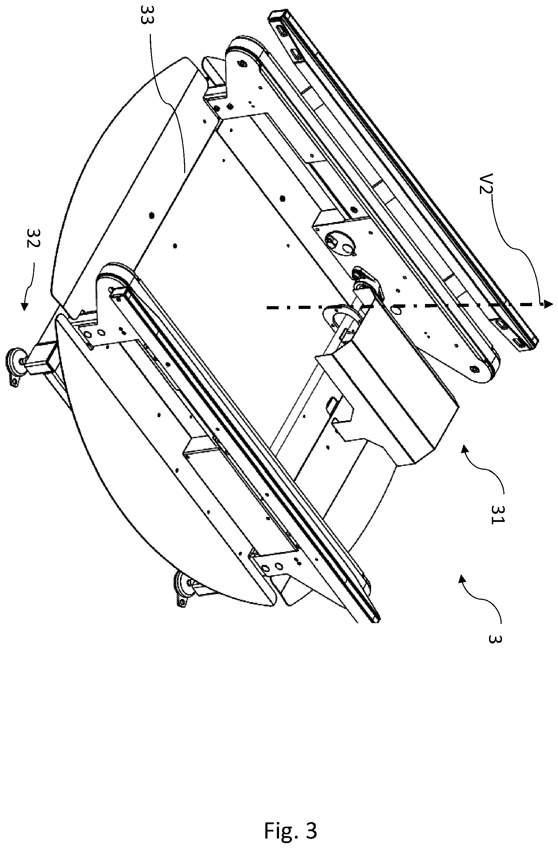

Referring to figure 3, which is a perspective view of an embodiment of a second rotation unit 3 of the system 1 according to the invention. The second rotation unit 3 comprises a conveyor unit 31. The conveyor unit 31 may be identical to the conveyor unit 21 of the first rotation unit 2 or differ from the conveyor unit of the first rotation unit 21. The conveyor unit 31 is connected to a rotational platform 32. The rotational platform 32 is capable of rotating around a second vertical axis V2. The rotation of the rotational platform 32 leads to a rotation of the conveyor unit 32 around the second vertical axis V2. The rotational platform 32 comprises height adjustable legs, for either lowering or raising the rotational platform 32 and the conveyor unit 31. Arranged in-between the rotational platform 32 and the conveyor unit 31 is a waste shield 33. The waste shield is arranged to protect the rotational platform 32 from waste dripping of containers and/or lids arranged or being arranged on the rack. The second rotation unit 3 is a modular unit and may be moved independently of other parts within the system 1.

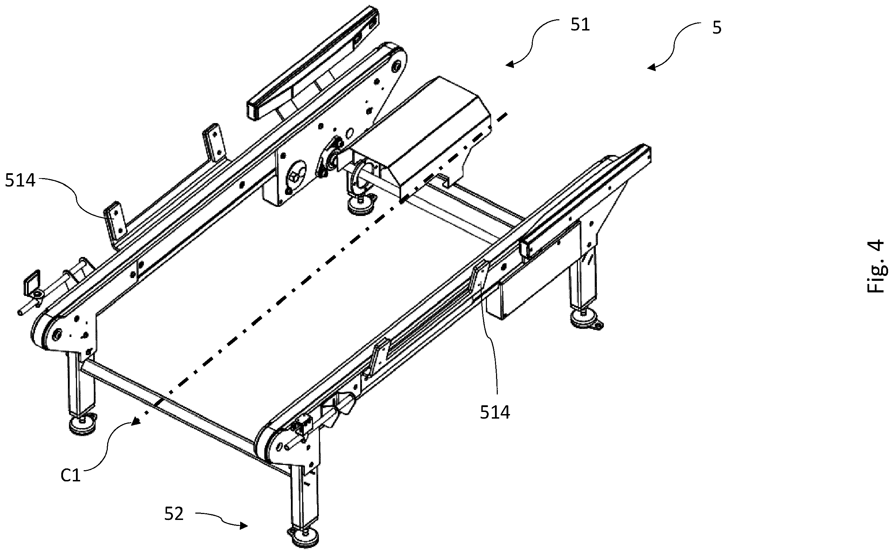

Referring to figure 4, which is a perspective view of an embodiment of a second outgoing unit 5 of the system 1 according to the invention. The second outgoing unit 5 comprises a conveyor unit 51. The conveyor unit 51 may be identical to the conveyor units 21, 31 of the first rotation unit 2 and/or the second rotation unit 3 or differ from the conveyor units 21, 31 of the first rotation unit 2 and/or the second rotation unit 3. The conveyor unit 51 of the second outgoing unit 5 is configured for receiving and conveying a rack along a first conveying direction C1. The conveyor unit 51 is connected to a platform 52. The rotational 52 comprises height adjustable legs, for either lowering or raising the platform 52 and the conveyor unit 51. The second outgoing unit 3 is a modular unit and may be moved independently of other parts within the system 1. The second outgoing unit 5 further comprises clamping means 514. The clamping means being in the form of opposing clamps 514 being configured to move into engagement with a rack received by the second out going unit 5. The opposing clamps 514 are configured to engage opposing sides of the rack to center the rack in-between the opposing clamps 514 and/or to re-orientate the rack. The opposing clamps 514 are moveable in a direction perpendicular to the first conveying direction C1. The centering of the rack may help in conveying the rack to one or more transport vehicles as consistent positioning when handing off the rack is achievable. In embodiments wherein the system only comprises a first outgoing unit 4 or more than two outgoing units 4 and 5, then any outgoing unit configured to convey to the rack to the one or more transport vehicles may comprise clamping means in the form of opposing clamps. Alternatively, an outgoing unit configured to convey to the rack to another outgoing unit may comprise clamping means in the form of opposing clamps.

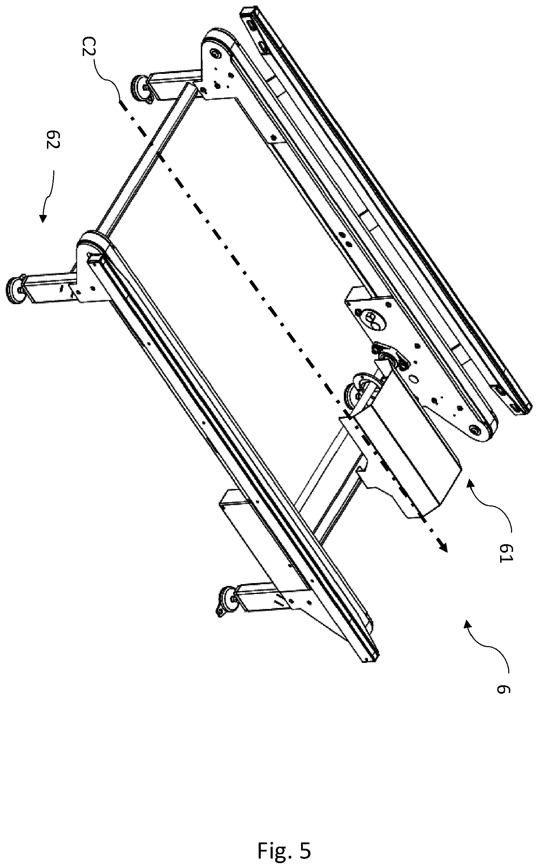

Referring to figure 5, which is a perspective view of an embodiment of a first ingoing unit 6 of the system 1 according to the invention. The first ingoing unit 6 comprises a conveyor unit 61. The conveyor unit 61 may be identical to the conveyor units 21, 31, 51 of the first rotation unit 2, the second rotation unit 3, and/or the second outgoing unit5or differ from the conveyor units 21, 31, 51 of the first rotation unit 2, the second rotation unit 3, and/or the second outgoing unit 5. The conveyor unit 61 of the first ingoing unit 6 is configured for receiving and conveying a rack along a second conveying direction C1.

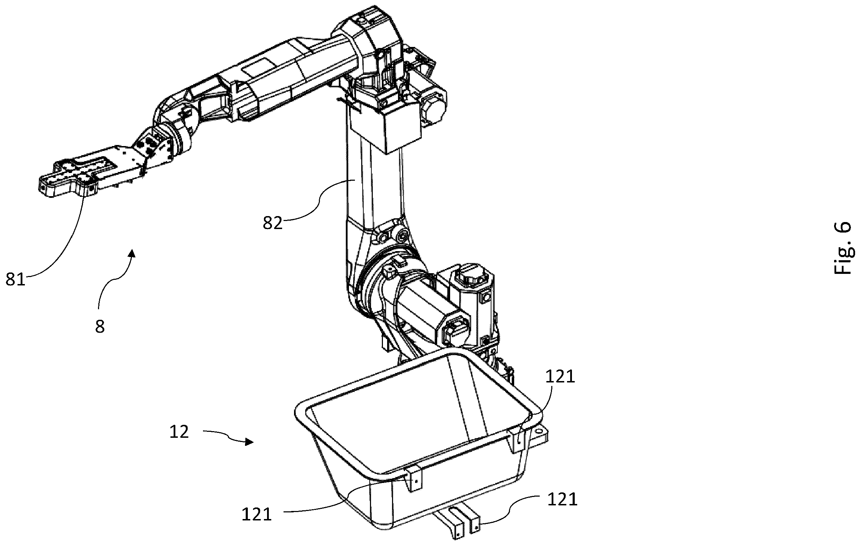

Referring to figure 6, which is a perspective view of an embodiment of a robotic arm 8 and a waste collector 12 of the system according to the invention. The robotic arm 8 comprises an arm 82. The arm 82 consists of several joints, which allow the arm to move both in the horizontal and vertical plane. The arm 82 may be mounted at a first end directly to a floor of a sterile processing department. The arm 82 being configured to move in-between a receiving position and a stacking position. At a second end of the arm 82 the robotic arm 8 comprises a lifting tool 81. When the arm 82 is in the receiving position, the lifting tool 81 is configured for engaging and holding a container and/or a lid conveyed to the robotic arm 81 by one or motorized conveyor elements 11. Subsequent to the lifting tool 81 engaging and holding the container and/or lid, the arm 82 is configured to move to the stacking position, thereby allowing the container and/or lid to be stacked on the rack. After the container and/or lid have been stacked onto the rack, the lifting tool 81 is configured to disengage from the container and/or lid and the arm 82 moves to the receiving position. The arm 82 may further be configured to move to a waste position. The waste position being a position allowing medical waste on the container and/or the lid to be collected in the waste collector 12. The collection of waste may happen by holding the container and/or lid above the waste collector for a period of tiem and let gravity move waste from the container and/or lid into the waste collector. The arm 82 is preferably configured to move from the receiving position to the waste position and then to the stacking position. The waste collector 12 may be a tub without or with a drain leading collected waste away. The waste collector 12 may be provided with one or more connection structures 121. The one or more connection structures may allow for the waste collector 12 to be connected directly to the one or more motorized conveyor elements 11. Alternatively, the waste collector 12 may be placed free standing onto a floor of a processing department.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. A system for arranging containers and container lids on a rack comprising a first rack side and a second rack side in a sterile processing department, said sterile processing department comprising one or more motorized conveyor elements configured for conveying the containers and container lids, and one or more transport vehicles configured for transporting the rack, where the system comprises:
• a first rotation unit for receiving the rack,
• a second rotation unit for conveying the rack, and
• a robotic arm configured to arrange containers and container lids conveyed by the one or more motorized conveyor elements onto the rack received by the first rotation unit,
wherein the first rotation unit is configured to:
- rotate the rack received relative to the robotic arm around a first vertical axis, thereby allowing the robotic arm to arrange containers on both the first rack side and the second rack side of the rack received, and
- convey the rack received to the second rotation unit
wherein the second rotation unit is configured to:
- rotate the rack received from the first rotation unit around a second vertical axis, and
- convey the rack received from the first rotation unit to one or more motorized conveyor elements configured for conveying the rack, or one or more transport vehicles configured for transporting the rack.

2. A system for arranging containers and container lids on a rack according to any of the preceding claims, the system further comprising a first outgoing unit, the first outgoing unit comprising one or more motorized conveyor elements, wherein the first outgoing unit is configured to:
- receive the rack conveyed by the second rotation unit, and
- convey the rack in a first conveyor direction to a second outgoing unit, or the one or more transport vehicles configured for transporting the rack.

3. A system for arranging containers and container lids on a rack according to any of the preceding claims, the system further comprising a first ingoing unit, the first ingoing unit comprising one or more motorized conveyor elements, wherein the first ingoing unit is configured to:
- receive the rack from a second ingoing unit or the one or more transport vehicles configured for transporting the rack.
- convey the rack in a second conveyor direction to the first rotation unit.

4. A system for arranging containers and container lids on a rack according to claims 2 and 3, wherein the first conveyor direction and the second conveyor direction are parallel to each other.

5. A system for arranging containers and container lids on a rack according to any of the preceding claims, the system further comprising a waste collector for collecting waste from the container and lids before being arranged on the rack.

6. A system for arranging containers and container lids on a rack according to any of the preceding claims, wherein the first rotation unit, and the second rotation unit, each comprises a conveyor unit for conveying the rack, and wherein each of the conveyor units are substantially identical.

7. A system for arranging containers and container lids on a rack according to claim 6, wherein each conveyor unit is a belt conveyor.

8. A system for arranging containers and container lids on a rack according to claim 7, wherein the belt conveyors of the first rotation unit and the second rotation unit comprises waste liners for shielding the respective belt conveyor from waste.

9. A system for arranging containers and container lids on a rack according to any of the preceding claims, wherein the first rotation unit and the second rotation unit each comprises a first cushion and a second cushion configured to delimit movement of the rack in a non-conveying direction, and where the first cushion and the second cushion are opposed to each other.

10. A system for arranging containers and container lids on a rack according to claim 9, wherein the first cushion and the second cushion each extends longitudinally with a first longitudinal end and a second longitudinal end, and wherein the first cushion and the second cushion taper toward their respective first longitudinal end and second longitudinal end.

11. A system for arranging containers and container lids on a rack according to any of the preceding claims, wherein the first rotation unit and the second rotation unit each comprises a rotational platform configured to rotate the rack, and wherein the first rotation unit and the second rotation unit each further comprises a waste shield, where the waste shield is arranged to be in-between the respective rotational platform and the rack when the first rotation unit and the second rotation unit receives the rack.

12. Use of a system according to any of claims 1-11 in a sterile processing department.

13. A sterile processing department comprising a system according to any of claims 1-11.

14. A sterile processing department according to claim 13, wherein the sterile processing department further comprises one or more motorized conveyor elements configured for conveying containers and container lids, one or more transport vehicles configured for transporting the rack, and a washing machine for washing containers and container lids on the rack, wherein the washing machine is configured for receiving the rack from the one or more transport vehicles.
